(19) Europäisches Patentamt
European Patent Office
Office européen des brevets

(11) **EP 4 349 260 A1**

(12) **EUROPEAN PATENT APPLICATION**

(43) Date of publication:
**10.04.2024 Bulletin 2024/15**

(21) Application number: **22199458.5**

(22) Date of filing: **04.10.2022**

(51) International Patent Classification (IPC):
**A61B 5/273** (2021.01)  **A61B 5/30** (2021.01)

(52) Cooperative Patent Classification (CPC):
**A61B 5/273; A61B 5/303;** A61B 2562/222

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
• **JOYE, Neil Francis**
**5656AG Eindhoven (NL)**
• **VAN KESTEREN, Hans Willem**
**5656AG Eindhoven (NL)**

(74) Representative: **Philips Intellectual Property & Standards**
**High Tech Campus 52**
**5656 AG Eindhoven (NL)**

(54) **PATIENT MONITOR COMPRISING ONE OR MORE UNIVERSAL PORTS**

(57) The present invention relates to a patient monitor (10) comprising one or more universal ports (12) each configured to connect a cable having a universal connector (36) fitting into a universal port (12) for providing a measurement signal from a subject (30) to the patient monitor (10), wherein the patient monitor (10) comprises a selection unit (14), a distribution network (16), an analog front end unit (18) and one or more processing units (20).

FIG.5

EP 4 349 260 A1

**Description**

FIELD OF THE INVENTION

The present invention relates to a patient monitor comprising one or more universal ports to connect a cable having a universal connector fitting into an arbitrary universal port.

BACKGROUND OF THE INVENTION

To acquire a subject's vital signs on a patient monitor, consumables such as electrocardiogram (ECG) lead sets, oxygen saturation (SpO2) sensors, end-tidal CO2 (EtCO2) sensors or blood pressure cuffs are used to interface the patient to the patient monitor. The main function of these consumables is to transmit the measurement signal (e.g. an electrical potential or current) to the patient monitor, where further signal processing is performed in order to determine and display the vital sign.

Recently, there is a trend to add signal processing into the consumables, including e.g. analog to digital conversion, and thus, making the consumables "smart".

SUMMARY OF THE INVENTION

It is an object of the present invention to provide a patient monitor allowing to use consumables easily and flexibly with the patient monitor.

In an aspect of the present invention a patient monitor is presented that comprises:

one or more universal ports each configured to connect a cable having a universal connector fitting into a universal port for providing a measurement signal from a subject to the patient monitor;

a selection unit configured to obtain cable information from the connected cable and to determine, based on the cable information, a type of cable of the connected cable and a type of measurement for which the connected cable is configured;

a distribution network configured to provide the measurement signal from the one or more universal ports to corresponding processing units either directly or via an analog front end unit based on the type of cable and the type of measurement;

an analog front end unit configured to convert an analog measurement signal from analog to digital and to provide the converted measurement signal to the distribution network; and

one or more processing units each configured to process the measurement signal based on the type of measurement and to output the processed measurement signal.

Preferred embodiments of the invention are defined in the dependent claims.

The present invention is based on the idea that entities such as e.g. hospitals will probably not completely switch from using standard (analog) cables to only using smart (digital) cables across their whole facility. Therefore, smart cables and standard cables will coexist. This makes assets management more challenging: A digital port will not support standard (analog) cables, and standard (analog) ports will not support smart (digital) cables. This may raise issues from a workflow and backwards compatibility perspective. It might be incomprehensible to a user that a smart cable can only be used on a digital port and that a standard cable cannot be connected to a digital port. Also, this could raise confusion and thus uneasiness among the hospital staff, which could in turn prevent a smooth adaption to a smart cable solution.

The present invention provides a patient monitor comprising one or more universal ports configured to connect a universal connector of a cable. A user (e.g. hospital staff) initializing one or more measurements on a subject (or patient, e.g. a human or an animal) with a patient monitor may plug a universal connector of a cable connected to a sensor deployed on the subject into any one of the universal ports, independently of the type of the cable (e.g. analog or digital) and the type of measurement (e.g. ECG, SpO2, etc.). Upon connecting the cable, the patient monitor may automatically obtain cable information from the connected cable and determine the type of cable and the type of measurement. Based on the measurement type, a measurement signal may be internally processed by a corresponding processing unit - if required - preceded by a conversion of a non-digital to a digital signal based on the type of cable. Processing a signal may comprise e.g. filtering of the measurement signal or artefact suppression etc. Then, the processed measurement signal may be outputted by the patient monitor, e.g. for visualization for the user to be interpreted.

The patient monitor according to the present invention comprises one or more universal ports that accept both analog (or "standard") cables and digital (e.g. smart) cables. Therefore, the present invention allows for easily and flexibly using cables of different types and/or for different types of measurements on the same universal port on the same patient monitor. For example, a smart (digital) and a standard (analog) cable each comprising a universal connector may be flexibly used on the same universal port (one after the other). Thus, the present invention helps to improve the handling of consumables with a patient monitor. Accordingly, the present invention supports an adaption to a smart cable solution.

Preferably, the selection unit is configured to determine whether the measurement signal obtained from the connected cable is an analog signal or a digital signal. Thus, the selection unit may determine the type of the cable by distinguishing between an analog measurement signal and a digital measurement signal. This may be done based on the cable information as well, which may indicate that the type of measurement indicates or requires an analog measurement or that the cable is an analog cable. The obtained measurement signal can thus be routed to the analog front end.

In one embodiment, the selection unit is further configured to obtain cable information comprising a cable identification, wherein the cable information is stored

in a memory provided in the cable; and/or
as an optically readable identification provided at the cable; and/or
on a radio frequency identification, RFID, tag or on a near field communication, NFC, tag provided in or at the cable.

Therefore, the present invention provides several options/alternatives for the selection unit to obtain the cable information from the cable, all ensuring a quick and reliable setup of one or more measurements with the patient monitor. For example, the optically readable identification may be a barcode or a quick response, QR, code. In other examples, the cable information may be stored in a memory or an NFC tag provided in the connector of the cable.

In another embodiment, the selection unit is further configured to determine the type of cable and/or the type of measurement based on the measurement signal. This is based on the idea that each measurement signal may have specific characteristics which may allow to distinguish one measurement signal from other measurement signals and that the characteristics of the measurement signal may also indicate or allow to determine the type of the connected cable. Therefore, the selection unit may determine the type of cable and/or the type of measurement, for example even if no cable information is obtained from the connected cable (e.g. if it cannot be read or if it incomplete or if it is not available at all). Thus, a quick and reliable setup of one or more measurements with the patient monitor may be ensured.

Preferably, the patient monitor further comprises a user interface configured to receive user input indicating the type of cable and/or the type of measurement. Therefore, the selection unit may determine the type of cable and the type of measurement even if no cable information is obtained from the connected cable. Thus, a quick and reliable setup of the measurements with the patient monitor is ensured.

In one embodiment, the selection unit is configured to determine a default value for the type of cable and/or the type of measurement if no cable information is obtained from the connected cable. The user interface may be configured to request a validation of the default value from the user. This way, the user may be informed about the issue of a default value and obtain a request for information on the type of cable and/or the type of measurement. The user may adapt the default value to the actual type of cable and/or the actual type of measurement. Thus, a quick and reliable setup of one or more measurement on a subject with the patient monitor may be ensured.

In another embodiment, the user interface is configured to request a validation of the type of cable and/or the type of measurement as determined by the selection unit from the user. This way, the user may approve or change the determined type of cable and/or the determined type of measurement upon request which may be a comfortable and quick way for the user to initialize one or more measurements while ensuring the correct setup of the one or more measurements with the patient monitor.

Preferably, the patient monitor further comprises one or more drive units to control one or more measurements performed on a subject, each drive unit being configured to be connected to a universal port based on one or more control signals from the selection unit. These drive units may support performing measurements on a subject. For example, the pulse length of an oxygen saturation (SpO2) measurement may be adjusted for skin-tone of the subject by a drive unit. The control signals provided by the selection unit may ensure that each measurement is supported by a corresponding drive unit suitable to support the performance of the specific measurement.

Preferably, the analog front end unit is further configured to amplify and/or filter the analog measurement signal. This way, the quality of analogous signals may be maintained/ensured, e.g. for long cables.

In an embodiment, the distribution network and/or the analog front end unit may comprise one or more switching elements to process the measurement signal based on one or more control signals obtained from the selection unit. As such, switching elements may allow to ensure that the measurement signal is processed correctly based on the determined type of cable and the determined type of measurement. For example, switching elements may allow to direct a measurement signal to a specific analog front end unit and/or specific processing unit. Thus, the switching elements may ensure that a specific type of measurement is processed by a specific processing unit and/or a specific analog front end unit.

In another embodiment, the patient monitor may further comprise an adapter comprising a first adapter port configured to connect to a non-universal connector of a cable and a second adapter port configured to connect to a universal port. The adapter may permit to use a cable comprising a specialized connector on the patient monitor. Thus, the adapter may help to avoid the necessity of redesigning a non-universal connector of a standard cable to fit into the universal port.

Preferably, the adapter further comprises an adapter analog front end circuitry, an analog-digital converter configured to convert an analog measurement signal to a digital measurement signal and a serializer configured to convert a first number of input signals of the digital measurement signal to a second number of output signals. The adapter comprising a serializer may allow to use a standard cable comprising a connector with more pins than supported by the universal port. The universal port may support for example eight pins and the serializer may convert a number of signals greater than eight, e.g. ten, to eight signals suitable for the universal port.

BRIEF DESCRIPTION OF THE DRAWINGS

These and other aspects of the invention will be apparent from and elucidated with reference to the embodiment(s) described hereinafter. In the following drawings:

Fig. 1 schematically shows a measurement on a subject according to an embodiment of the present invention.
Fig. 2 shows a schematic diagram of an embodiment of a patient monitor according to the present invention.
Fig. 3 shows a schematic diagram of an embodiment of a patient monitor according to the present invention comprising a user interface.
Fig. 4 shows a schematic diagram of an embodiment of a patient monitor according to the present invention comprising one or more drive units.
Fig. 5 shows a schematic diagram of another embodiment of a patient monitor according to the present invention.
Fig. 6 shows a schematic diagram of an embodiment of a distribution network as e.g. used in the patient monitor shown in Fig. 5.
Fig. 7 shows a schematic diagram of another embodiment of a patient monitor according to the present invention.
Fig. 8 shows a schematic diagram of an embodiment of a distribution network as e.g. used in the patient monitor shown in Fig. 7.
Fig. 9 schematically shows an embodiment of an analog front end unit according to the present invention.
Fig. 10 shows a schematic embodiment of a distribution network according to the present invention.
Fig. 11 shows another embodiment of an analog front end unit according to the present invention.
Fig. 12 shows a schematic diagram of an embodiment of a patient monitor according to the present invention comprising an adapter.
Fig. 13 shows a schematic diagram of an embodiment of the adapter as e.g. used in the patient monitor shown in Fig. 12.

DETAILED DESCRIPTION OF EMBODIMENTS

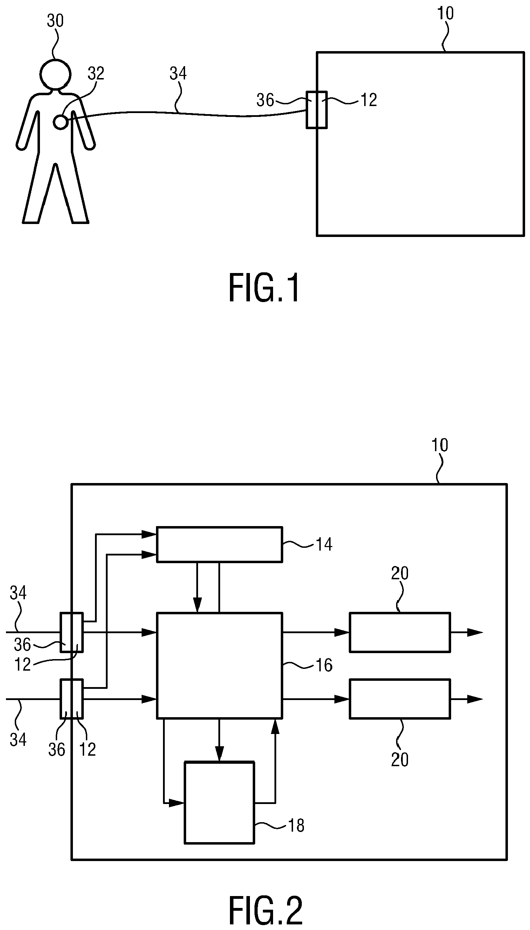

Fig. 1 schematically shows a measurement on a subject 30 with a patient monitor 10 according to an embodiment of the present invention. A sensor 32 (e.g. an ECG lead set, an SpO2 sensor or an EtCO2 sensor, etc.) is deployed on subject 30 for acquiring/obtaining a measurement signal. Cable 34 is configured to transmit the measurement signal from sensor 32 to the patient monitor 10 via universal connector 36 fitting into the universal port 12. The measurement signal (e.g. a vital sign) may then be processed by the patient monitor 10 and displayed on the patient monitor 10.

Fig. 2 shows a schematic diagram of an embodiment of a patient monitor 10 comprising two universal ports 12 according to the present invention. However, the patient monitor 10 may generally comprise any number of universal ports 12, i.e., one or more universal ports 12.

Usually, consumables (such as ECG sensors etc.) used for measurements on subjects (e.g. in a hospital) may comprise different connectors (e.g. with different numbers of pins) and/or different types of cables (e.g. configured to transmit analog or digital data). The ports of a patient monitor are generally specialized on one type of cable and one type of measurement. Therefore, a cable has to be connected to a corresponding, specific port.

As the number of ports in a patient monitor is usually limited (e.g. by the size of the patient monitor) only a certain number of different measurement types with a specific type of cable may be connected to the patient monitor. Also, it may lead to incomprehension to a user (e.g. hospital staff) why a digital cable will not fit into an analog port (a port configured to connect to analog cables) and why an analog cable will not fit into a digital port (configured to connect to digital cables) although the port may support the same type of measurement performed on the subject.

The present invention allows the use of analog cables and digital (e.g. smart) cables on any one of (generally) multiple universal ports 12 provided at the patient monitor 10. This allows to handle consumables used for measurements on subjects more flexibly and more easily while ensuring the correct setup of the measurements with the patient monitor.

The patient monitor 10 according to the present invention may comprise one or more universal ports 12 each configured to connect a cable having a universal connector 36 fitting into a universal port 12 for providing a measurement signal from a subject 30 to the patient monitor 10. A user initializing the one or more measurements may connect the universal connector of a cable connected to a sensor 32 deployed on the subject 30 to any one of the universal ports 12, independently of a type of cable (e.g. analog or digital) and a type of measurement (e.g. ECG, SpO2, etc.). Upon connecting the cable, the patient monitor 10 may automatically obtain cable information from the connected cable and determine the type of cable and the type of measurement. Based on the measurement type, the measurement signal may be internally processed by a corresponding processing unit - if required - preceded by a conversion of non-digital to digital signals based on the type of cable. The processed measurement signal may be outputted, e.g. for visualization for a user. Therefore, the present invention allows for easily and flexibly using cables of different types and/or for different types of measurements on the same universal port 12 on the same patient monitor 10.

The term "universal" refers to the capability of the universal port to accept different types of cables and/or to accept cables used to perform different types of measurements on a subject.

The term "port" refers to a place on/at the patient monitor where cables may be connected to via the connector of the cable, wherein the port is configured to obtain electrical information (e.g. a measurement signal, cable information from a memory etc.) from the connected cable.

The term "analog cable" refers to a cable configured to transmit analog information, in particular analog measurement signals, to the patient monitor.

The term "analog port" refers to a port configured to connect an analog cable.

The term "digital cable" refers to a cable configured to transmit digital information, in particular digital measurement signals, to the patient monitor.

The term "smart cable" refers to a cable configured for signal processing including analog to digital conversion. This includes cables which are configured to perform an analog to digital conversion of information (e.g. an analog measurement signal), e.g. in the connector of the cable.

The term "digital port" refers to a port configured to connect to a digital cable.

The term "consumables" refers to one or more sensors used for a certain measurement (e.g. ECG, SpO2, blood pressure etc.) and one or more cables comprising a connector, wherein the one or more sensors and the one or more cables comprising the connector are configured to transmit a measurement signal, e.g. a vital sign obtained from a sensor deployed on a subject for the measurement, to a port of a patient monitor.

The term "setup" refers to the initialization of a measurement on a subject with the patient monitor, comprising connecting a connector of one or more cables to the (universal) port of the patient monitor and the patient monitor determining the correct type of cable and the correct type of measurement in order to process the measurement signal accordingly.

The patient monitor 10 shown in Fig. 2 comprises a selection unit 14, a distribution network 16, an analog front end (AFE) unit 18 and one or more processing units 20.

The selection unit 14 is configured to obtain cable information from the connected cable and to determine which type of measurement and which type of cable is connected to each universal port 12. The patient monitor 10 may generally comprise any number of universal port 12, i.e. one or more universal ports 12. For each universal port 12, the selection unit 14 may determine if the cable is an analog ("standard") cable or digital (e.g. smart) cable, and which type of measurement (e.g. ECG, SpO2, EtC02 etc.) is performed. Based on the type of cable and the type of measurement, the selection unit 14 may provide one or more control signals for the distribution network 16 and the AFE unit 18. The selection unit 14 may further provide one or more control signals for the one or more processing units 20 (control signals for the processing units 20 are not shown in Fig. 2).

The distribution network 16 may connect each universal port 12 to the corresponding processing unit 20 either directly or via the analog front end (AFE) unit 18. The selection may be based on one or more control signals defined by the selection unit 14. The AFE unit 18 may perform analog-to-digital conversion for each analog measurement signal, i.e. measurement signals from analog ("standard" or "conventional") cables. The AFE unit 18 may comprise switches controlled by one or more control signals from the selection unit 14 to connect a universal port 12 - and thus the measurement signal - to the corresponding internal circuit of the AFE 18. In one embodiment according to the present invention, the AFE unit 18 may be further configured to perform front-end signal processing in order to amplify and/or filter the analog measurement signal.

The one or more processing units 20 may be any kind of means configured to process one or more measurement signals. A processing unit 20 may be for example a microcontroller or microprocessor. A processing unit 20 per each universal port 12 may process (e.g. filtering, artefact suppression, etc.) the measurement signal, if necessary, proceeded by an analog-to-digital conversion by the analog front end (AFE) unit 18. In one embodiment, the processing unit 20 may transmit the processed signal to a monitor display (the patient monitor may e.g. comprise a monitor display) for visualization or an external device (e.g. mobile device, command centre, electronic medical record (EMR) etc.).

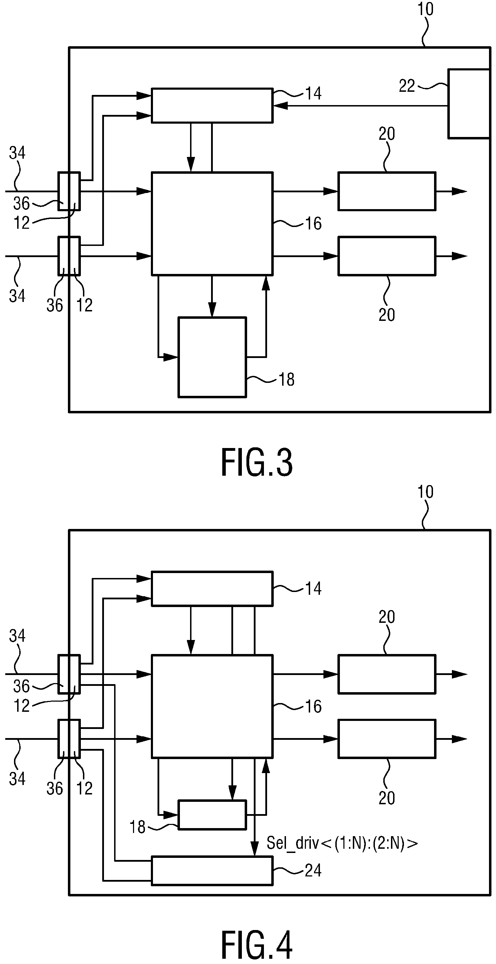

Patient monitor 10 may further comprise a user interface 22, e.g. as shown in the embodiment shown in Fig. 3, and/or one or more drive units 24, e.g. as shown in the embodiment shown in Fig. 4.

The patient monitor 10 may further comprise one or more non-universal ports. For example, the one or more non-universal ports may support measurements (sometimes regarded as core measurements), such as oxygen saturation (SpO2), electrocardiogram (ECG), non-invasive blood pressure (NIBP), etc., wherein the one or more universal ports 10 may support e.g. additional measurements such as spot check temperature, end-tidal (EtCO2) measurement, electroencephalogram (EEG), etc. Using both universal and non-universal ports in a patient monitor may thus help to introduce universal port and smart cables into the market, which may take time to be used for all different kinds of measurements.

Fig. 3 shows a schematic diagram of the embodiment of patient monitor 10 shown in Fig. 2, further comprising a user interface 22 by which a user may provide information (user input) to the patient monitor 10. For example, the user interface 22 may be graphical user interface (GUI), e.g. a touchscreen. The selection unit 14 may be configured to obtain a user input from the user interface 22. The user input may comprise the type of cable and/or the type of measurement. This way, the selection unit 14 may determine the type of cable and the type of measurement based on the obtained cable information and/or the user input. For example, if no cable information is obtained from the connected cable, the selection unit 14 may determine the type of cable and the type of measurement from the user input provided by the user interface 22.

Fig. 4 shows a schematic diagram of the embodiment of patient monitor 10 shown in Fig. 2, further comprising one or more drive units 24. Drive unit 24 may comprise dedicated driver circuitry for an (analog) measurement supported by the patient monitor 10.

The individual driver circuits may be connected to the corresponding universal port 12 by switches (no switches are shown in Fig. 4) controlled by one or more control signals defined by the selection unit 14 based on the determined type of measurement and/or determined type of cable. The switches may be controlled for example by control signal Sel_driv<i:N>, wherein N is the number of (analog) measurements supported by the patient monitor 10 and i corresponds to a signal number (e.g. signal 1 or 2).

Patient monitor 10 may comprise one or more drive units 24 in order to support performing certain measurements on a subject. A universal port 12 may support for example eight pins: (i) power supply, (ii) ground/shield, (iii) 1-wire (for cable identification) and (iv) data lines. Even if the connector of the cable comprises the same number of pins, in this example eight pins, these e.g. eight pins of the connector 36 might not all be compatible with the universal port 12. For example, an SpO2 sensor comprising two LEDs (light emitting diodes) may need two power lines and dedicated driver electronics to perform the measurement. Thus, driver electronics provided in one or more drive units 24 may allow to perform certain measurements, for example such as the described SpO2 measurement.

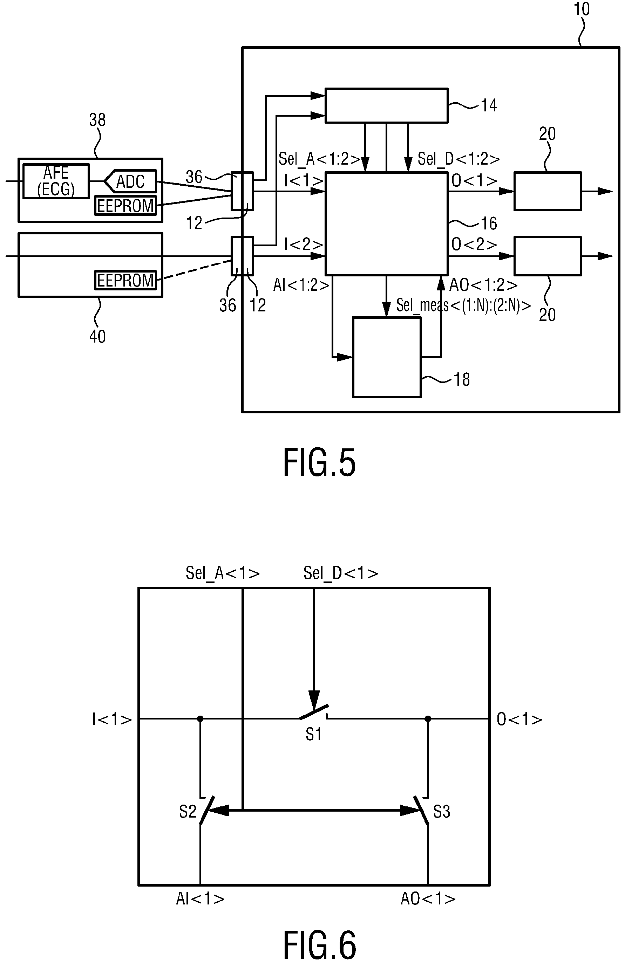

Fig. 5 shows an example of an embodiment of the patient monitor 10 shown in Fig. 2. In the example shown in Fig. 5, one digital cable 38 and one analog cable 40 are each connected to a universal port 12. As shown in Fig. 5, both the analog cable 38 and the digital cable 40 comprise a connector 36 fitting into the universal port 12. The digital cable 38 may contain a smart cable dongle implementing electronics, i.e. an analog front end (AFE) circuitry for the specific measurement, an analog-to-digital converter (ADC), and a memory device such as e.g. an electrically erasable programmable memory (EEPROM) that may store a cable identification of the digital cable 38. The digital cable 38 may further comprise a signal processing unit such as microcontroller or a microprocessor (not shown in Fig. 5). The analog cable 40 may or may not contain a memory device such as an EEPROM.

Each universal port 12 may be connected to a dedicated (corresponding) signal processing unit 20 (e.g. microcontroller, microprocessor) via the distribution network 16 - and if required - via the analog front end (AFE) unit 18. The main role of the processing units 20 is to process the measurement signals (e.g. filtering, artefact suppression, etc.) and transmit them, e.g. to a patient monitor screen (the patient monitor comprising a monitor screen) for visualization or to an external device (e.g. mobile device, command center, electronic medical record (EMR) etc.).

In case the cable comprises a cable identification stored in a memory device, e.g. such as an EEPROM, the selection unit 14 may obtain the cable identification stored in the memory device. The selection unit 14 may determine, based on the obtained cable identification which type of measurement and/or which type of cable is connected to each universal port 12. Thus, for each universal port 12, the selection unit 14 may determine if the cable is an analog (standard, conventional) cable or a digital (e.g. smart) cable, and which type of measurement (e.g. ECG, SpO2, etc.) is performed. The cable information may be also stored for example on a barcode or QR code at the cable/connector of the cable, or on a RFID or NFC tag provided in/at the cable etc.

Preferably, the selection unit 14 may also receive input from the user interface 22 of the patient monitor 10 (e.g. the monitor screen) such that the user can select or input the type of cable and the type of measurement for each universal port 12. This option may be needed in cases where the cable and/or the connector of the cable does not provide cable identification.

The selection unit 14 may provide three control signals, for example defined as follows:
A first control signal Sel_A<1:P> is indicating for each measurement signal if the measurement is analog ("standard cable"), wherein P is the number of universal ports 12, and wherein the first control signal Sel_A<1:P> comprises a vector comprising P entries. In Fig. 5, two universal ports 12 are shown, i.e. P = 2 and thus Sel_A<1:2>, which comprises the two sub-signals Sel_A<1> and Sel_A<2>. The first control signal may be input for the distribution network 16, as shown in Fig. 5. It should be pointed out, that the first signal may also be input to the processing unit 20 (not shown in Fig. 5).

A second control signal Sel_D<1:P> is indicating for each measurement signal if the measurement is digital (e.g. smart cable), wherein P is the number of universal ports 12, and wherein the second control signal Sel_D<1:P> comprises a vector comprising P entries. In Fig. 5, two universal ports 12 are shown, i.e. P = 2 and thus Sel_D<1:2>, which comprises the two sub-signals Sel_D<1> and Sel_D<2>. The second control signal may be input for the distribution network 16, as shown in Fig. 5. It should be pointed out, that it may also be input to the processing unit 20 (not shown in Fig. 5).

A third control signal Sel_Meas<P:N> is indicating the type of measurement (e.g. ECG, SpO2, etc.), wherein P is the number of universal ports 12 and N is the number of (analog) measurements supported by the patient monitor, and wherein the third control signal Sel_Meas<P:N> comprises a matrix comprising P x N entries. The third control signal may be input for the AFE unit 18, as shown in Fig. 5. It should be pointed out, that may also be input to the processing unit 20 (not shown in Fig. 5).

The selection unit 14 may provide any number of control signals and/or control signals of any type suitable to control the processing of one or more measurement signals obtained from one or more connected cables.

Fig. 6 shows an embodiment of a distribution network 16 as it may be used e.g. in the embodiment of the patient monitor 10 shown in Fig. 5. Only one "channel", i.e. one universal port 12, is shown in Fig. 6. The input signal I<1> is connected to a universal port 12 with the number 1. The output signal O<1> is connected to a corresponding processing unit 20. Signal AI<1> may be an input signal to the analog front end (AFE) unit 18 and the signal AO<1> may be an output signal of the AFE unit 18. The distribution network 16 may comprise three switches S1, S2 and S3 controlled by a first control signal Sel_A<1> and a second control signal Sel_D<1>. In the case of a digital measurement signal, when the second control signal Sel_D<1> is equal to 1, switch S1 is closed, thereby directly connecting the input signal I<1> to the output signal O<1>. In this situation, the first control signal Sel_A<1> is equal to zero, which leaves the switches S2 and S3 open. In the case of an analog measurement signal, when the first control signal Sel_A<1> is equal to one, the switches S2 and S3 are closed, thereby directly connecting the input signal I<1> with the input signal AI<1> for the AFE unit 18 and connecting the output signal AO<1> of the AFE unit 18 with the output signal O<1>. In this situation, the second control signal Sel_D<1> is equal to zero, which leaves switch S1 open. The selection unit 14 may thus ensure that equation

$$Sel\_A < i > = \overline{Sel\_D < i >} \qquad\qquad \text{Equation (1)}$$

is valid, wherein i is the channel number corresponding to the universal port number, i.e., Sel_D<1> is the inverse of Sel_A<i>.

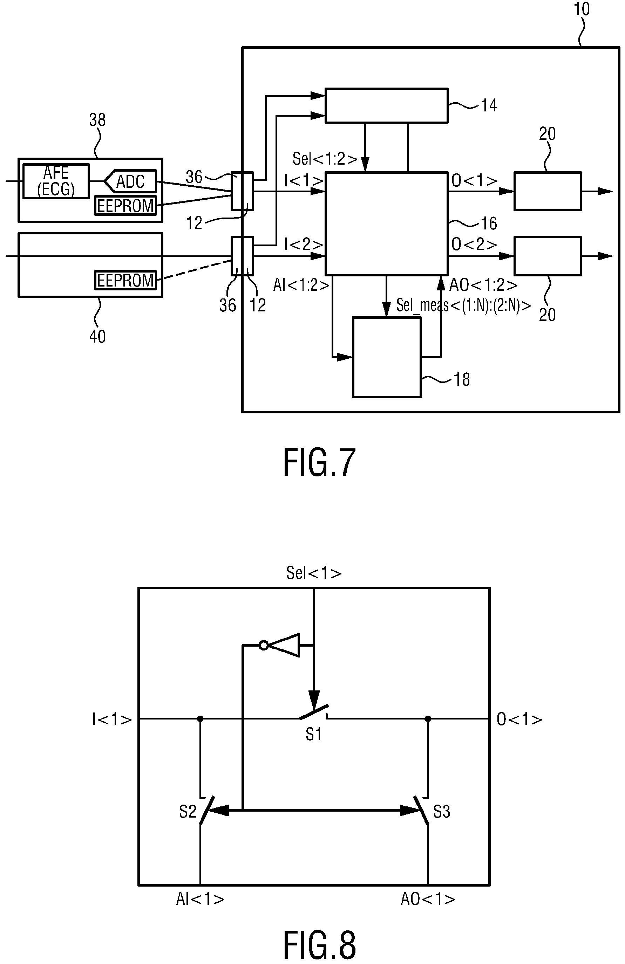

Fig. 7 shows a schematic diagram of another embodiment of a patient monitor 10 according to the present invention. In Fig. 7, one smart (digital) cable 38 and one analog (standard) cable 40 are each connected to a universal port 12. However, patient monitor 10 may generally comprise any number of universal ports 12, i.e. one or more universal ports 12. As an alternative to the embodiment shown in Fig. 5, two control signals provided by the selection unit 14 (Sel_A<1:P> and Sel_D<1:P> in Fig. 5) may be replaced by one control signal (Sel<1:P> in Fig. 7). As shown in Fig. 7, the selection unit 14 may provide one instead of two control signals for the distribution network 16, compared to the embodiment shown in Fig. 5. Fig. 8 shows an embodiment of a distribution network 16 for one universal port 12 which may be used with the embodiment of the patient monitor 10 shown in Fig. 7.

Fig. 8 shows a schematic diagram of an embodiment of a distribution network 16 which may be used e.g. in the embodiment of the patient monitor 10 shown in Fig. 7 but for one universal port 12. It should be noted that the distribution network 16 may generally be configured for any number of universal ports 12, i.e. for one or more universal ports 12.

The distribution unit 16 shown in Fig. 8 comprises the three switches S1, S2 and S3 for directing one measurement signal from one universal port 12. The control signal Sel<1> is applied to a first switch S1. The signal is also inverted, by an inverter, and applied to the second switch S2 and the third switch S3. Equation (1) may thus always be met since it is implemented in the circuity of the distribution network 16.

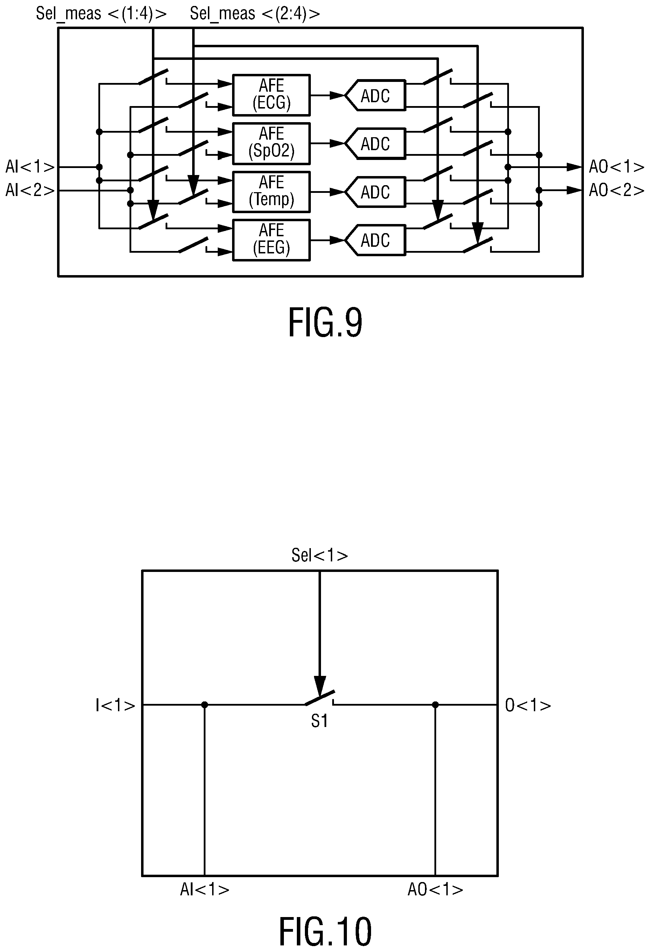

Fig. 9 shows an embodiment of an analog front end (AFE) unit 18 according to the present invention. In Fig. 9, only two "channels", i.e. two universal ports 12, are depicted. However, the patient monitor 10 may generally comprise any number of universal ports 12, i.e. one or more universal ports 12. As mentioned above, the input signal AI<i> of the AFE unit 18 (i.e. A<1> and A<2>) and the output signal AO<i> of the AFE unit 18 (i.e. AO<1> and AO<2>) are connected to the distribution network 16, as shown in Fig. 5 and Fig. 7.

The analog front end (AFE) unit 18 may contain N number of analog front end circuits and analog digital converters (ADC), N being the number of (analog) measurements supported by the patient monitor 10. In this example, N = 4 since the AFE unit 18 supports in this example ECG, SpO2, temperature and EEG (or work of breathing, WoB).

Each AFE circuit may be designed specifically for one type of measurement.

Each input signal AI<i> to the AFE unit 18 may be connected to no AFE circuit (in case the cable is a digital (smart) cable and thus is not connected to the AFE unit 18) or to one AFE circuit via the corresponding switches (in case the cable is an analog cable). These switches are controlled by the control signal Sel_meas<(P:N)>, wherein P is the number of universal ports 12 and N is the number of supported measurements by the patient monitor 10.

The same control signals may ensure that a corresponding ADC is connected to the correct output signal AO<i>. The selection unit 14 may also ensure that an AFE circuit does not have more than one input (i.e. more than one AI<i> signal).

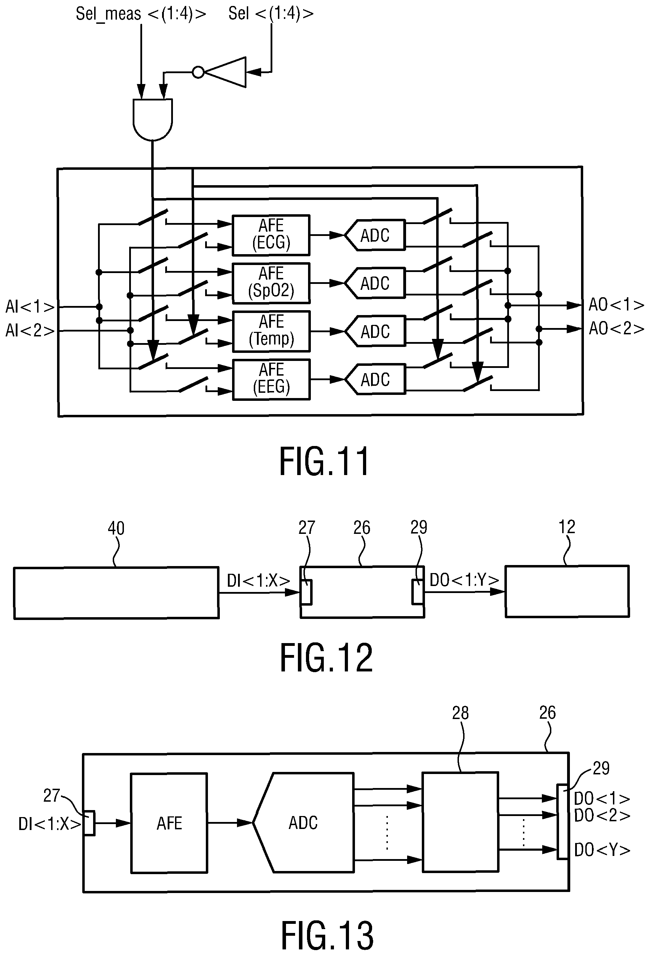

Fig. 10 shows a schematic embodiment of a distribution network 16 according to the present invention. If the control signal Sel_meas<P:N> are set such that an input signal A<i> to the AFE unit 18 is not connected to any analog front end (AFE) circuit (i.e. all switches for this "channel" remain open) when a digital cable is connected the corresponding universal port 12 (e.g. see embodiment shown in Fig. 11), the distribution network 16 can be simplified as shown in Fig. 10. In this case, switches S2 and S3, as shown in Fig. 6 and Fig. 8, are not required.

For example, if a digital (e.g. smart) cable is connected to universal port 12 with the number 1 (corresponding to "channel 1"), switch S1 is closed such that the input signal I<1> corresponds to the output signal O<1>, i.e. I<1> = O<1>, and the control signal (for the case where N=4) Sel_meas<1:4> = (0, 0, 0, 0), such that all switches connecting the input signal AI<1> to the AFE unit 18 to each AFE circuit are open. On the other hand, if an analog cable is connected to the universal port 12 with the number 1 (corresponding to "channel 1"), switch S1 is controlled to be open, and the measurement signal obtained from the universal port with the number 1 is connected to the AFE unit 18. For example, for channel 1, the control signal is Sel_meas<1:4> = (0, 1, 0, 0), thus indicating a SpO2 measurement, wherein the input signal AI<1> to the AFE unit 18 is connected to the corresponding AFE circuitry for a SpO2 measurement signal (see Fig. 10).

Fig. 11 shows an embodiment of an analog front end unit 18 according to the present invention. The embodiment in Fig. 11 represents an alternative option to ensure that control signals Sel_meas<P:N> are correctly set, namely implemented in hardware. The additional circuitry comprising a NOT and an AND gates, as shown in Fig. 11, may be implemented either in the selection unit 14 or in the analog front end (AFE) unit 18. The additional circuitry implements:

$$Sel\_meas < (i:n) > \ AND \ \overline{Sel < (i:n) >} \qquad \text{Equation (2)}$$

wherein i is the channel number corresponding to the universal port number and n is the measurement number (e.g. n=2 for SpO2). This embodiment e.g. may allow for the embodiment of the distribution network 16 shown in Fig. 10.

Figs. 12 schematically shows an embodiment of a patient monitor 10 further comprising an adapter 26. Fig. 13 schematically shows an embodiment of the adapter 26 shown in Fig. 13. The embodiments shown in Fig. 12 and Fig. 13 is based on the idea that if the cable comprises a connector having more pins than supported by the universal port 12, the cable may not be able to be used with the universal port 12. A possible solution is to use an adapter 26 comprising a first adapter port 27 configured to connect to a non-universal connector of a cable 40 and a second adapter port 29 configured to connect to a universal port 12. Cable 40 comprising a non-universal connector is connected to the first adapter port 27, and the second adapter port 29 is connected to the universal port 12 of the patient monitor 10.

Adapter 26 may comprise an analog front end (AFE) circuitry, an analog digital converter (ADC) and a serializer 28 that is configured to convert a first number of signals X received by the analog cable to Y signals supported by the universal port 12, wherein X is in general larger than Y.

The adapter 26 may have X input signals, i.e. the number of pins of the cable. These input signal DI<1:X> to the adapter 26 may feed into the AFE circuity of the specific measurement (e.g. ECG). After analog to digital conversion, the X signals may be provided as input to a serializer circuit that converts the X signals into an output signal DO<1 :Y> of the adapter 26 as an input to the patient monitor 10, wherein Y may be a number of signals supported by the patient monitor. For example, X may be 10 and Y may be 8. In another example, the adapter converts the X input signals such that a serial data stream for one or more data receive lines of the universal port 12 are provided.

This implementation provides two main advantages: (i) analog cables comprising a connector with more pins than supported by the universal port 12 can nevertheless be used with the patient monitor 10, and (ii) the non-universal connector of these analog cables does not need to be redesigned to fit the universal port 12.

The adapter 26 may be used to connect any type of analog cable, independently of the number of pins (even the ones with a number of pins equal or smaller than the number of pins supported by the universal port 12), to the universal port 12. Thus, this embodiment may provide a solution to connect analog cables having more pins than supported by a universal port 12 to the universal port 12.

In summary, the present invention helps to improve the handling of consumables (such as e.g. ECG lead sets, SpO2 sensors, end-tidal CO2 sensors or blood pressure cuffs) with a patient monitor. For this purpose, the patient monitor comprises one or more universal ports each configured to connect a cable having a universal connector. For example, digital (e.g. smart) cables and analog ("standard" or "conventional") cables comprising a universal connector may be flexibly used on the same universal port. The patient monitor may automatically determine the type of the cables connected to the patient monitor and/or the type of measurement performed on a subject. Therefore, a user (e.g. hospital staff) may easily and flexibly setup one or more measurement on a subject with the patient monitor while the correct setup of the measurement is ensured.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

**Claims**

1.  A patient monitor (10) comprising:

    one or more universal ports (12) each configured to connect a cable having a universal connector (36) fitting into a universal port (12) for providing a measurement signal from a subject (30) to the patient monitor (10);
    a selection unit (14) configured to obtain cable information from the connected cable and to determine, based on the cable information, a type of cable of the connected cable and a type of measurement for which the connected cable is configured;
    a distribution network (16) configured to provide the measurement signal from the one or more universal ports (12) to corresponding processing units (20) either directly or via an analog front end unit (18) based on the type of cable and the type of measurement;
    an analog front end unit (18) configured to convert an analog measurement signal from analog to digital and to provide the converted measurement signal to the distribution network (16); and
    one or more processing units (20) each configured to process the measurement signal based on the type of measurement and to output the processed measurement signal.

2.  Patient monitor (10) as claimed in claim 1,
    wherein the selection unit (14) is configured to determine whether the measurement signal obtained from the connected cable is a digital signal or an analog signal.

3.  Patient monitor (10) as claimed in any one of the preceding claims,

    wherein the selection unit (14) is configured to obtain cable information comprising a cable identification, wherein the cable information is stored
    in a memory provided in the cable; and/or
    as an optically readable identification provided at the cable; and/or
    on a radio frequency identification, RFID, tag or on a near field communication, NFC, tag provided in or at the cable.

4.  Patient monitor (10) as claimed in any of the preceding claims,
    wherein the selection unit (14) is further configured to determine the type of cable and/or the type of measurement based on the measurement signal.

5.  Patient monitor (10) as claimed in any one of the preceding claims,
    further comprising a user interface (22) configured to receive user input indicating the type of cable and/or the type of measurement.

6.  Patient monitor (10) as claimed in claim 5,
    wherein the selection unit (14) is configured to determine a default value for the type of cable and/or the type of measurement if no cable information is obtained from the connected cable.

**EP 4 349 260 A1**

7. Patient monitor (10) as claimed in claim 6,
wherein the user interface (22) is configured to request a validation of the default value from the user.

8. Patient monitor (10) as claimed in claims 5 to 7,
wherein the user interface (22) is configured to request a validation of the type of cable and/or the type of measurement as determined by the selection unit (14) from the user.

9. Patient monitor (10) as claimed in any one of the preceding claims,
further comprising one or more drive units (24) to control one or more measurements on the subject (30), each drive unit (24) being configured to be connected to a universal port (12) based on one or more control signals from the selection unit (14).

10. Patient monitor (10) as claimed in any one of the preceding claims,
wherein the analog front end unit (18) is further configured to amplify and/or filter the analog measurement signal.

11. Patient monitor (10) as claimed in any one of the preceding claims,
wherein the distribution network (16) and/or the analog front end unit (18) comprises one or more switching elements to process the measurement signal based on a control signal obtained from the selection unit (14).

12. Patient monitor (10) as claimed in any one of the preceding claims,
further comprising an adapter (26) comprising a first adapter port configured to connect to a non-universal connector of a cable and a second adapter port configured to connect to a universal port (12).

13. Patient monitor (10) as claimed in claim 12,
wherein the adapter (26) further comprises:

an analog front end circuitry;
an analog-digital converter configured to convert an analog measurement signal to a digital measurement signal; and
a serializer (28) configured to convert a first number of input signals of the digital measurement signal to a second number of output signals.

10

FIG.1

FIG.2

FIG.3

Sel_driv<(1:N):(2:N)>

FIG.4

FIG.5

FIG.6

FIG.7

FIG.8

Sel_meas <(1:4)>    Sel_meas <(2:4)>

AI<1>

AI<2>

AFE (ECG)    ADC

AFE (SpO2)    ADC

AFE (Temp)    ADC

AFE (EEG)    ADC

AO<1>

AO<2>

FIG.9

Sel<1>

I<1>    S1    O<1>

AI<1>    AO<1>

FIG.10

**FIG.11**

**FIG.12**

**FIG.13**

Europäisches
Patentamt

European
Patent Office

Office européen
des brevets

# EUROPEAN SEARCH REPORT

Application Number

EP 22 19 9458

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2015/009315 A1 (DRAEGER MEDICAL SYSTEMS INC [US]) 22 January 2015 (2015-01-22) <br> * page 2, lines 32-34 * <br> * page 3, line 10 - page 4, line 3 * <br> * page 7, lines 9-15 * <br> * page 14, lines 14-29 * <br> * page 15, lines 25-27 * <br> * page 17, lines 4, 5, 21-34 * <br> * page 18, lines 11-24 * <br> * page 21, lines 6-8 * <br> * figures 4B, 5 * <br> ----- | 1-13 | INV. <br> A61B5/273 <br> A61B5/30 |
| X | US 2011/209915 A1 (TELFORT VALERY G [CA] ET AL) 1 September 2011 (2011-09-01) <br> * paragraphs [0089], [0116], [0125], [0176], [0198], [0206] * <br> * figure 19 * <br> ----- | 1,3-5, 10,11 | |
| A | US 2005/113704 A1 (LAWSON COREY J [US] ET AL) 26 May 2005 (2005-05-26) <br> * paragraphs [0007], [0022], [0025] * <br> ----- | 1-13 | TECHNICAL FIELDS SEARCHED (IPC) <br><br> A61B |

The present search report has been drawn up for all claims

| Place of search | Date of completion of the search | Examiner |
|---|---|---|
| The Hague | 15 March 2023 | Worms, Georg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.......................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 19 9458

This annex lists the patent family members relating to the patent documents cited in the above-mentioned European search report.
The members are as contained in the European Patent Office EDP file on
The European Patent Office is in no way liable for these particulars which are merely given for the purpose of information.

15-03-2023

| Patent document cited in search report | | Publication date | Patent family member(s) | | Publication date |
|---|---|---|---|---|---|
| WO 2015009315 | A1 | 22-01-2015 | NONE | | |
| US 2011209915 | A1 | 01-09-2011 | US 2011209915 A1 | | 01-09-2011 |
| | | | WO 2011047211 A1 | | 21-04-2011 |
| US 2005113704 | A1 | 26-05-2005 | NONE | | |

EPO FORM P0459

For more details about this annex : see Official Journal of the European Patent Office, No. 12/82